# EUROPEAN PATENT APPLICATION

(11) **EP 1 564 554 A1**
(43) Date of publication of application: **17.08.2005**
(21) Application number: 04075439.2
(22) Date of filing: 12.02.2004
(51) Int. Cl.: G01N 33/50

(54) **Method for the detection of early B cell populations in vaccine development**

(71) Applicant: PEPSCAN SYSTEMS B.V., 8219 PH Lelystad (NL); Universiteit Utrecht Holding B.V., 3584 CL Utrecht (NL)
(72) Inventor: Akresteijn, Gerardus Josephus Adrianus, 3533 VJ Utrecht (NL); Hensen, Everard Johannes, 3581 HH Utrecht (NL); Scibelli, Antonio, 80147 Napoli (IT); van der Most, Robbert Gerrit, 3984 CL Odijk (NL); Meloen, Robert Hans, 8231 AP Lelystad (NL); Turkstra, Jouwert Anne, 8252 HA Dronten (NL)
(74) Representative: Prins, Adrianus Willem, Mr. Ir.

(57) **Abstract**

The present invention discloses an affinity-binding assay comprising a particle having at least four copies of a target molecule and at least two binding molecules specific for said target molecule, wherein a first of said binding molecules is associated with a first label and a second of said binding molecules is associated with a second label, wherein a particle having said first label and a particle having said second label are distinguishable from a particle having both said first and said second label, wherein said first and said second binding molecule each comprise at least two binding regions specific for said target molecule.

The present invention also discloses a composition comprising a first binding molecule associated with a first label and a second binding molecule associated with a second label, wherein the signal obtained from said first label and said second label is distinguishable from the combined signal of said first and second label, characterised in that said first and said second binding molecule each comprise at least two binding regions specific for essentially the same target molecule, preferably for essentially the same epitope on said target molecule and a method for selecting a synthetic antigen from a collection of at least three antigens comprising using the disclosed composition and method.

The invention further discloses an antigen obtainable by above described method and capable of inducing an early immune response, a kit of parts to perform said method, the use of antigens selected by said methods for use as a vaccine, and the use of antibodies and antigens as a medicament.

## Description

The invention relates to the field of immunology and vaccine development. The invention further relates to the detection of early B cell responses after immunisation with specific antigens.

Vaccines have proven to be extraordinary effective means to improve public health (Jackson *et al*., 2002). However, conventional vaccine development can be hampered by technical problems in pathogen inactivation as well as high costs.

Furthermore, many antigens are not readily recognised by the immune system as foreign and consequently, they elicit no adequate immune response. In these cases, peptide-based synthetic vaccines are valid alternatives (Jackson *et al*., 2002). Peptide vaccination has been successfully used to prevent infectious diseases (Monzavi-Karbassi *et al*,. 2002),and to treat tumours (Ribas *et al*,. 2003; Noguchi *et al*,. 2003), amyloidosis (Nicoll *et al*,. 2003) and autoimmune disease (Liu *et al*,. 2002).

One difficulty in preparing vaccines against antigens that are not recognised by the body as foreign is caused by the phenomenon of tolerance. Normally, an immune response is elicited against antigens that are recognised as foreign, but not against self-antigens. In order for a vaccine to be successful, it must be sufficiently foreign. Only when a vaccine is foreign enough i.e. sufficiently immunogenic will the immune system respond to the vaccine and an immune response is induced. Conversely, however, the immune cells and/or antibodies must still be capable of recognizing the self-substance or the tolerated antigen, and thus the vaccine cannot be too "foreign". For this reason, peptides of said antigens are made, and said peptides are modified to enhance the immunogenicity of said antigens. Said peptides or modified peptides are used as a peptide vaccine.

The development of a peptide vaccine is a labour-intensive and time-consuming process. Peptide vaccine candidates must elicit an immune response and cause clonal expansion of specific T cells and/or B cells. Said immune response is elicited by epitopes that are recognized by T cells and/or B cells and initiate a proliferative reaction of antigen specific T and B cells. Said proliferative reaction syn.: "clonal expansion" starts within a few hours after the initial contact of an antigen with the immune system, for example after vaccination. The number of specific immune B cells increases by clonal expansion until high levels of effector cells are reached after three or four weeks after vaccination. The levels of these effector cells after clonal expansion are high enough for conventional methods to detect the effector cells. To detect whether peptides expose B or T cell epitopes, screening of a large set of peptides in animal immunization procedures is necessary. Because the immune repertoire of B cell and T cell specificities is very diverse (Davis and Bjorkmann, 1988), and the frequency of antigen-specific B cells participating in an antibody response is very low, detection of B cells presents a major difficulty in its functional and molecular characterization (Newman *et al.,* 2003), and poses a major problem for investigators studying B-cell reactions. After clonal expansion, activated B cells produce antigen specific antibodies (Adams *et al*., 2003). Therefore, B cell response against an antigen is usually measured by measuring the level of antigen specific antibodies in circulating blood. As a result, a large number of experimental animals has to be kept for a considerable amount of time, because after normal immunization procedures it takes at least 4 to 5 weeks before antibodies, directed against an antigen have reached levels at which they can be compared and be interpreted. In effect, this provides a major obstacle for fast and systematic synthetic peptide vaccine development.

B cells can be detected specifically by direct or indirect staining methods, usually achieved by coupling antigens or antibodies to a signalling molecule, like for example a fluorescent stain (fluorescein isothyocyanate, FITC, or Rhodamine), or a chemical, radioactive, or enzymatic signal or another marker substance that enables a skilled person to detect binding of the marker substance to a B cell. Many methods have been developed to increase the sensitivity of B cell detection to a level where small numbers of antigen specific B cells can be detected in a large collection of cells, like for example in spleen cells. Until now, the numbers of antigen specific B cells before the process of clonal expansion were too low to be detected by exiting detection methods. Said clonal expansion process increases the numbers of B cells to a level where either B-cells or antibodies produced by said B cells, can be detected. Based on fluorescent staining technology, flow cytometry was developed and further adapted to detect antigen specific B cells (McHeyzer-Williams *et al.*, 1993). Historically, investigators have employed and combined two different approaches to study antigen specific B cells. One approach is hybridoma technique to capture immune reactive B cells and the other approach is to study genetically manipulated animals with an enhanced number of antigen specific B cells. Both methods are too insensitive to detect the low levels of emerging antigen specific B cells after vaccination before clonal expansion has been completed.

One method developed for the detection of B cells has been adapted from the staining of T cells. The sensitivity of detecting T cells was enhanced by incubating the cells with a tetrameric peptide-MHC molecule that binds to T cell receptors specific for said peptide-MHC complex (Altman *et al.*, 1996; Stetson *et al.*, 2002). Although the above-mentioned method has been adapted for the detection of antigen-specific B cells (Newman *et al.*, 2003), the sensitivity of the normal procedures was only increased tenfold. This is still far too insensitive for effective detection of emerging low- frequency antigen specific B cells, shortly after vaccination.

Another method to enhance the detection level of low frequency target cells has been described by Townsend *et al*., 2001, who succeeded in lowering the detection threshold by staining B cells with two reagents specific for the same B cell receptor, that had been conjugated to two different labels (dubbed 'single epitope multiple staining'). The principle of this sequential staining procedure (in which the first reagent is used at sub-saturating concentrations) is that the probability of false positive cells binding *both* reagents is much smaller than the probability of false positive cells binding only one of the reagents. However, Townsend *et al.* only characterized adoptively transferred BCR-transgenic cells, leaving the question unanswered whether their methods could be extrapolated to the analysis of bona fide immune responses, including those induced by vaccines.

This "single epitope multiple staining procedure" reduced the background staining, thereby enabling an increase in the sensitivity of flow cytometry by 1 or 2 orders of magnitude enabling the detection of high affinity antigen-specific B cells in vivo. This increase in sensitivity may enable the detection of transgenic B cells or B cells after clonal expansion is completed, but it is still too insensitive for the detection of low frequency early B cells within 7 days after immunization. Furthermore, the method is complicated and the authors warn the public that it is critical to note that several requirements must be met before this strategy will be effective in increasing the sensitivity of flow cytometry. The above-described method of Townsend *et al.* used as an antigen a foreign antigen for mice namely chicken egg white lysozyme. When applied to the detection of specific B cells against a self-antigen like GnRH, the method could not discriminate any specific B cells from the background staining (see example 1 of the working examples in this application). Therefore, the method of Townsend *et al* was not suited for the detection of low-frequency B cells against self-antigens.

We have developed a highly sensitive and reproducible affinity-binding assay to select peptide vaccine candidates based on specific staining of emerging B cells during the clonal expansion at a very early stage of the immune response. An affinity-binding assay is a test for detecting or measuring binding of two or more substances that have a certain affinity for each other. This can for example be the binding between an antibody and an antigen, or of an enzyme to its substrate, or of a hormone to its hormone receptor. In the present application we describe, as an example, the affinity binding between the antigen specific B cell receptor to an antigen. Of course, said affinity binding may be detected and measured by various methods. As an example, we disclose the detection of affinity binding by an adapted flow cytometry method.

The present application discloses a binding assay and a test method using this binding assay in which a particle or a cell comprising at least four target molecules, for example membrane bound antibodies or receptors or antigen-specific B cell receptors (BCRs), can be detected by contacting said particle or cell with at least two binding molecules that have a different label. Detecting of particles or cells by flow cytometry is based on staining said particle or cell with a fluorescent label and detecting labelled particles or cells by registering fluorescence signals using the fluorescence detectors. Each fluorescent label has its own characteristics for intensity and the level of background staining. This application discloses that staining with a molecule comprising a multiple form of an antigen, in which at least two, or three, preferably four, or more, binding epitopes of essentially the same antigen are present and exposed, increases the intensity of the staining. A further increase in sensitivity is achieved by combining the staining of above described multiple forms of an antigen with two different fluorescent labels. For this purpose, two quantities of essentially the same multiple antigen molecules are associated with two different labels. In this case, labels are selected that differ in their fluorescent signal, like for example R-phycoerythrin (PE)-labelled neutravidin and allophycocyanin (APC) coupled to streptavidin. By contacting the cells with said both differently labelled antigenic molecules, the cells bind both molecules and combine the two different fluorescent labels on the surface. This contacting can for example be done in consecutive steps, for example by first incubating the cells with the first fluorescent-labelled antigen molecule in a sub-saturating amount, followed by incubation with the second fluorescent-labelled antigen in a more saturating or a saturated amount. Another example of a way of staining particles or cells with both fluorescent-labelled antigen molecules is by mixing the two differently labelled antigen molecules at an approximately equimolar amount and incubating the particles or cells with the mixture. Because the chance of aspecific binding of two separate molecules on the same particle is very small, the specificity of the method is increased. This increased specificity, combined by the higher avidity of the binding with multiple antigen molecules, causes a surprising increase in sensitivity and allows for the specific detection of only 20 to 30 cells in one million cells. In this application, the antibody molecules or the antigen-specific B cell receptors present on the surface of the immune cells are called the target molecules. The antigen molecules that bind to said target molecules are in this application named binding molecules. Said binding molecules may comprise one or more peptides or proteins or fragments thereof. In addition said binding molecules may comprise a staining molecule. A particle in this application can be a virus or a microorganism, or a part of a cell or yeast. Preferably, the size of said particle is at least about the size of a virus and at most about the size of a thousand cells, more preferably about the size of a hundred cells, even more preferably about the size of 10 cells, even more preferably about the size of one cell. In this patent application we use the phrase "distinguishable" for the fact that by interpreting the results of preferably flow cytometry, double stained particles or cells can be distinguished from particles or cells that have only one label or no label at all. Preferably this distinction is made through distinction of the signal from said first and second label. Label means in this context preferably a fluorescent staining label.

Therefore, the present invention discloses an affinity-binding assay comprising a particle having at least four copies of a target molecule and at least two binding molecules specific for said target molecule, wherein a first of said binding molecules is associated with a first label and a second of said binding molecules is associated with a second label, wherein a particle having said first label and a particle having said second label are distinguishable from a particle having both said first and said second label, wherein said first and said second binding molecule each comprise at least two binding regions specific for said target molecule. Said particle can also be a cell, therefore the invention also discloses the above-described assay, wherein said particle comprises a cell. Said cell may be a living cell or said cell may be treated with a fixative such as for example fixatives that are normally used in flow cytometry, like for example formalin, acetone, alcohol, or glutaraldehyde.

Because the method of the invention is very sensitive, this is the first time that such a method is capable of detecting very low frequency antigen specific B cells, like for example very early immune B cells. These very early immune cells are only present at very low frequency because these cells have not yet accomplished the complete clonal expansion process. Therefore, the present invention discloses an affinity-binding assay as described above, wherein said cell comprises an activated immune cell in the clonal expansion phase of a primary immune response. In mice for example, this activation takes place within hours after first or second contact with an antigen and results in antibody levels in peripheral blood within three to four weeks.

The method is especially suitable for the detection of very low frequency B cells and saves a lot of time because one does not have to wait any more for the antibody response to evolve. This shortens the animal phase for each peptide from at least 3 to 4 weeks to one week. Furthermore, the detection of very low frequency B cells is also very suitable for the detection of memory B cells. This may be important for assessing the immune status against certain diseases. A B cell in this application means a B cell in any state of activation or differentiation, including for example antigen specific precursor B cells, antibody secreting cells, plasma cells and memory B cells. Therefore, the present invention discloses in a preferred embodiment the assay as described above, wherein said cell comprises a B cell. The possibilities to count and phenotypically characterize B cells as disclosed in this application enable a person skilled in the art to measure the early immune response against immunization or infection, e.g., proliferation and differentiation of naive B cells into antibody secreting cells, memory B cells and plasma cells.

Because the combination of said multiple antigen molecules with the multiple staining technique increases the sensitivity surprisingly much, the present invention discloses an affinity-binding assay as described above, wherein said particle having said first and said second label increases the sensitivity of the affinity-binding assay.

The binding properties of said multiple antigen molecules are increased with the number of exposed antigenic sites on the molecule. In this application, tetramer molecules comprising four antigenic sites have been tested as an example. Of course other numbers of antigens repeated in the same molecule may also have the desired effect. Therefore, the present invention discloses an affinity-binding assay as described above, wherein said first and said second binding molecule each comprise at least two binding regions specific for said target molecule. Preferably said number of binding regions is four.

The binding may be increased even further if the antigenic sites or epitopes of said target molecule are essentially identical epitopes. Therefore, the present invention discloses an affinity-binding assay as described above, wherein said at least two binding regions are essentially identical. A big advantage of the assay is that the binding molecules of the detection assay may represent an epitope of a native protein, because in this way, immunization with altered antigens of said native protein can be monitored for the cross-specificity for said native protein. A native protein is in this application a protein as it is present in nature. Therefore, the present invention discloses an affinity-binding assay as described above, wherein said binding molecule represents an epitope of a native protein. The present invention is very suited for detecting low frequency B cells. Said B cells, when activated by contact with an antigen, expose during their clonal expansion an antigen-specific B cell receptor or BCR on their cell surface. Suitable B cell receptors are for example the well-known cell bound antibody-like molecules that are inserted in the wall of the B cell with their Fc fragment and which are specifically recognising antigen with the variable region, said antigen being the antigen that was used to activate the B cells (Kouskoff *et al*., 2000). In the described assay, said BCRs are the target molecules with which the binding molecules bind. Therefore, the present invention discloses an affinity-binding assay as described above, wherein said target molecule comprises a BCR.

Even more preferably the assay detects binding to the antigen specific site of the BCR. Therefore, the present invention discloses an affinity-binding assay as described above, wherein said target molecule comprises a variable region of said BCR.

The assay is performed with a set of binding molecules detecting the target molecule. Said binding molecule preferably comprising tetramer molecules. Therefore, the present invention in another embodiment discloses a composition comprising a first multiple binding molecule associated with a first label and a second multiple binding molecule associated with a second label, wherein the signal obtained from said first label and said second label is distinguishable from the combined signal of said first and second label, wherein each binding molecule comprises at least four binding regions specific for essentially the same target molecule, preferably for essentially the same epitope on said target molecule.

Now that there is an assay and a composition for detecting and selecting B cells at a very early stage during clonal expansion, there is no need any more to wait for at least 3 to 4 weeks for antibody levels before an antigen can be evaluated for vaccine purposes. This enables a person skilled in the art to select in a fast and reliable way an antigen from a large collection of antigens. In another embodiment, relating to therapeutic vaccination procedures involving, for instance, tumor antigens and hormones, individual responses can be monitored and non-responsive individuals can be identified more rapidly.

To overcome the tolerance of the immune system for certain antigens, a range of modified antigens can be produced, for example by the production of synthetic peptides. Changes in the peptides may increase the immunogenicity, and by testing said peptides, new and improved antigens may be detected and selected by the assay and the method of the invention. A range of changes made to a peptide in order to changes the antigenicity of said peptide results in various forms of said peptide, which is also called a collection of peptides. Antigen-specific B cell detection as disclosed in this application expedites the evaluation of vaccine immunogenicity, and enables analysis of the fine-specificity of the response. For instance, the precise (sub)-serotype specificities of the novel generation of multivalent conjugate vaccines can now be analysed in unprecedented detail. Therefore, the present invention discloses a method for selecting a synthetic antigen from a collection of at least two antigens comprising using the composition as described above for detecting in an affinity-binding assay for immune cells specific for said synthetic antigen, clonal expansion of antigen specific immune cells in samples of cells obtained from a mammal immunized with an antigen of said collection of antigens, and comparing said clonal expansion with the clonal expansion of another mammal immunized with another antigen form said collection and selecting from said collection an antigen with which clonal expansion was more extensive than clonal expansion observed with at least one other antigen from said collection. A more extensive clonal expansion means in this application that the numbers of specific B cells increase faster and to higher levels compared to clonal expansions as reaction to other antigens. In mice for example, increased clonal expansion at 7 days is indicative for an increased clonal expansion at 14 days and usually for a high level of antibodies later in the immune response. In rats or other animals, this increase may show later or earlier in the immune response but be highly indicative of a more extensive clonal expansion, if compared to the clonal expansion in other rats or other animals immunized with another antigen of said collection of antigens.

Of course, the above-described method may use any of the affinity-binding assays as are described above. Therefore, the present invention discloses a method for selecting a synthetic antigen as described above, wherein said affinity-binding assay comprises any assay as described herein.

Of course, for said synthetic peptides to be a good vaccine against a native protein, cross reactivity of the antibodies directed against said synthetic peptide antigen with the native protein from which said peptides were derived is highly appreciated. Therefore, the method as described above is preferably used wherein said at least two binding molecules comprise as a binding part for said target molecule a synthetic antigen of said collection of antigens, or a native antigen or homologue of said antigen. A native antigen is in this application an antigen as it occurs in a protein in nature. A homologue of an antigen is a substance showing the same antigenic characteristics in kind, not necessarily in amount. Said homologue may comprise a natural peptide or protein or a synthetic peptide or a part thereof. Therefore, direct B cell staining procedures enable the assessment of specific B cell receptor cross-reactivity between peptide vaccines and the native antigens from which they are derived.

The invention also discloses the method as described above, further comprising selecting an antigen for which clonal expansion of antigen-specific immune cells more extensive than the clonal expansion with at least two other antigens.

With the assay and the method as described above B cells specific for a certain antigen can be selected and isolated. Said isolated B cells may also be further tested for the specificity and avidity of their binding to the native protein, also called the native antigen or homologue of said antigen. Therefore, the invention teaches a method as described above further comprising evaluating whether said antigen-specific immune cells are specific for a native antigen or homologue of said antigen.

The above described methods and assays enable a person skilled in the art to select and isolate antigen-specific cells. Therefore, the invention discloses a method for an affinity-binding assay for selecting antigen specific immune cells, comprising: (a) contacting a cell having at least four copies of a target molecule with at least two binding molecule, preferably tetrameric binding molecules, said binding molecules specific for said target molecule, wherein a first of said binding molecules is associated with a first label and a second of said binding molecules is associated with a second label and; (b) detecting cells staining with each label and; (c) selecting cells binding both labels.

The present invention teaches with above described methods a new way to test and select a synthetic antigen from a collection of antigens inducing a cross-reactive immune response with a native protein. This is an important improvement of the technical possibilities in this field and opens the possibility for new methods of selection and thus to other products. Therefore, the invention discloses a synthetic antigen, inducing a cross-reactive immune response with a native protein, selectable with a method as described above for use as a vaccine.

To increase the antigenicity of a peptide, said peptide motif can be made into dimer or trimeric or multimeric peptides wherein two or three or more peptides are linked directly to each other or through a spacer molecule. Combining two identical peptides into a dimer peptide further increases the immunogenic properties of said peptide. Therefore, the present invention discloses an immunogenic peptide, obtainable by above described method, wherein said peptide comprises a dimer peptide.

In said dimer peptide, the peptides can be linked by sulphur bridges or by a linkage molecule. Another method of increasing the immunogenicity of a peptide is by combining a peptide in a tandem peptide and/or a tandem dimer peptide. From WO 96/40755 it is known that the tandem-dimer principle applied to a variant of the GnRH-I molecule resulted in a vaccine that was highly effective in low doses and with a mild adjuvant. Therefore, the present application discloses a method for selecting an immunogenic peptide derived from an antigen comprising detecting clonal expansion of antigen specific immune cells as described above, wherein said peptide comprises a tandem dimer peptide.

The antigenic properties of peptides provided herein are further optimised using a variety of techniques, such as replacement-net mapping, allowing detecting peptides with improved characteristics. For example one such peptide may be a peptide that binds to an antibody directed against an epitope, thereby mimicking the immunogenic properties of said epitope. Such a peptide is called a mimotope. This method has been disclosed in patent application WO 00/29851. Therefore, the present application also discloses an immunogenic peptide derived from a protein, comprising a mimotope of said protein.

Amino acid substitution of at least one amino acid by a D-amino acid and/or a L-alanine as described in WO 02/22659 also increases the immunogenicity of a peptide. Therefore, the present invention discloses an immunogenic peptide derived from a protein, wherein at least one amino acid is substituted by a D-amino acid and/or a L-alanine.

One example of a peptide hormone for which peptides have been prepared according to the sequence of said hormone and which are tested in abovementioned method is gonadotrophin releasing hormone or GnRH. Therefore, the application discloses abovementioned methods, wherein said antigen comprises a GnRH-peptide hormone and/or part thereof or a GnRH-derived peptide. Gonadotrophin releasing hormone (GnRH) is a decapeptide, produced by the hypothalamus. The GnRH travels through portal circulation to the pituitary to stimulate the release of gonadotrophins FSH and LH (Talwar, 1999). Anti-GnRH immunization blocks the fertility of both male and female animals (Meloen *et al*., 2001), and GnRH vaccines have applications in prostate and in female cancer (Talwar, 1999; Fuerst *et al.*,1997). To compare the usefulness of the different methods, mice were immunized with GnRH-like peptides conjugated to ovalbumin (OVA). Three different peptides were produced, i.e., GnRH-monomer (pEHWSYGLRPGC) GnRH-tandem (pEHWSYGLRPGQHWSYGLRPGC) and GnRH tandem dimer (TDK, the dimerized form of pEHWSYkLRPGQHWSYkLRPGC in which 'k' represents a D-lysine residue). The GnRH tandem and TDK peptide conjugated to a carrier protein have shown to be highly immunogenic, resulting in GnRH neutralizing antibodies and a full biological effect in all treated animals (Meloen *et al.,* 1994; Oonk *et a*. 1998). Therefore, the present application provides an immunogenic peptide obtainable by the above-described method, wherein said peptide is derived from GnRH.

Another example of a peptide hormone for which peptides have been prepared according to the sequence of said protein and which are tested in abovementioned method is Gastrin. Therefore, the application discloses an immunogenic peptide derived from a peptide hormone and obtainable by above- mentioned methods, wherein said peptide is derived from Gastrin. Gastrin is a peptide hormone that is important in the regulation of acid secretion and the growth of both normal and malignant gastrointestinal epithelium (Jensen *et al.*, 2002). Tumor cells can respond both to circulating endocrine gastrin (Watson *et al*., 1989) and locally produced gastrin, which acts in an autocrine or paracrine manner (Hoosein *et al.*, 1990). Effective inhibition of gastrin may be beneficial as a therapy for colorectal cancer, gastric cancer and pancreatic cancer, as gastrine acts as a growth factor for these tumours. Gastrine receptor antagonists are not fully effective because several receptor subtypes are involved in the action of gastrin and high amounts are required to displace gastrin. Neutralisation of gastrin may circumvent this problem.

Peptides derived from gastrine can be used for vaccination against gastrin (amino acid sequence pEGPWLEEEEEAYGWMDF). Suitable peptides for vaccination include amino acid 1-X, wherein X = 5 to 12. A Cysteine is included at the N- or C-terminal end for conjugation purposes. The peptides can also be applied in tandem or tandem dimer formulation with a Cysteine added at the N- or C-terminus or a cysteine between the two gastrin sequences in the tandem, as in pEGPWLEEEECQGPWLEEEE. L-Lysines or D-Lysines are introduced in the tandem formulation to allow dimerisation of these peptides via the cysteine and conjugation via the L- or D-Lysine, like in 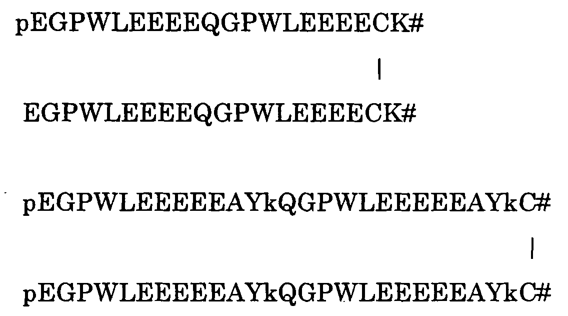 in which 'k' represents a D-lysine residue.
These peptides were tested as a vaccine in mice and at 7 and 14 days after immunization specific B cells were detected with the method of the invention using tetramer Gastrin peptide molecules. The following peptide was used to form a tetramer: pEGPWLEEEEEAYGWMDFK($)# , wherein # is amide; pE is pyroGlutamine; and K($) is biotinylated Lysin

Therefore, the present invention discloses an immunogenic peptide obtainable by any of the above-described methods wherein said peptide is derived from Gastrin.

Yet another example of a hormone for which peptides have been prepared is Human Chorionic Gonadotropin-beta (hCG-β), which is normally produced by the placenta during pregnancy (Meyer *et al.*, 2001). The protein can be detected in serum or urine. hCG beta is elevated during the first trimester of pregnancy and is often used as an indicator of pregnancy. hCG- β is also a tumor-associated antigen that is in various types of cancer. Most commonly, hCG beta is elevated, >10 mIU/ml (Gauchi *et al*., 1981), in gynaecological cancers (Gauchi *et al.*, 1981; Higashida *et al*., 2001; Kinugasa *et al*., 1992), but also in colorectal (Lundin *et al*., 2000), seminoma testicular (Hara *et al*., 2002), bladder (Hotakainen *et al*.,2002), liver (Szavay *et al.,* 2000), stomach (Bateman *et al.*, 1995), pancreas (Syrigos *et al*., 1998), lung (Uckaya *et al.*, 1998), brain (Fujimaki *et al.,* 2000), and kidney (Hotakainen *et al*., 2002) cancers. Non-malignant elevations may be observed in pregnancy (Meyer *et al*., 2001), ulcers (Manabe *et al*., 1985), duke's disease (Carpelan-Holmstrom *et al*., 1996), and cirrhosis (Hoermann *et al.*, 1992). Levels of hCG- β are useful in monitoring the effectiveness of treatment, such as chemotherapy, against tumor progression. Cancer patients with elevated levels of hCG- β have significantly shorter survival time than patients with levels below the median level. Effective inhibition of hCG- β is, therefore, beneficial as a therapy in gynaecological, colorectal, seminoma testicular, bladder, liver, stomach, pancreas, lung, brain, and kidney cancers, both in the prevention of the disease and in advanced disease. Effective inhibition of hCG- β is, therefore, also beneficial as a therapy in ulcers, duke's disease, and cirrhosis. The accession number of the hCG- β sequence is: P01233 . The Swiss-Protein entry name is: CGHB_HUMAN. The hormone comprises 165 amino acids, wherein amino acid 1-20 form a signal peptide

Overlapping 20-mer peptides were used for immunization with a N-terminal Cystein for conjugation to a carrier protein. The following peptides were included: 21-40, 31-50, 41-60, 51-70, 61-80, 71-90, 81-100, 91-110, 101-120, 111-130,121-140, 129-165 and 141-165

Yet another hormone for which peptides have been prepared is Human Parathyroid hormone-related protein (PTHrP).

Hypercalcaemia is one of the most common metabolic complications in patients with cancer. Hypercalcaemia occurs in a majority of the epithelial cancers in an advanced stage and is caused by PTHrP secretion by the tumor. PTHrP levels are undetectable in healthy persons. PTHrP secreted by tumours causes high calcium levels because it acts on the common PTH receptor.

PTHrP plays an important role in breast cancer. Approximately 50% of the primary tumours secrete PTHrP. This feature is associated with development of bone metastases. In these metastases PTHrP induces osteolytic bone lesions and is responsible for destruction of bone tissue. This osteolytic bone resorption results in release of TGF-B from the bone tissue, which in turn stimulates the production of more PTHrP

There are 2 types of hypercalcaemia in cancer:

Humoral hypercalcaemia wherein primary tumours are producing PTHrP, which acts as a hormone and after being secreted into the bloodstream it acts on bone and kidney to increase calcium levels.

Local osteolytic hypercalcaemia: PTHrP also plays a role in bone metastases. It can induce a local osteolysis near a bone metastasis, allowing the tumour metastasis to grow into the bone tissue. In breast cancer almost all metastases contain high levels of PTHrP whereas only 17% of the non-bone metastases expresses PTHrP. It causes fractures and bone-pain in these patients. The current treatment for hypercalcaemia is administration of bisphosphonates, which reduces calcium levels. But this treatment does not affect the PTHrP levels.

Neutralisation of PTHrP in cancers by vaccination against PTHrP may contribute to an improved quality of life of patients with advanced cancer regression. The Swiss-Prot entry name is: PTHR_HUMAN. The accession number of the sequence is P12272. The length of human PTHrP is 177 amino acids 1-177, wherein amino acids 1 - 36 comprise a leader sequence.

Effective peptides are N-terminal peptides of the PTHrP fragment 37-70, including 37-X, wherein X = 47 to 56. A C-terminal Cysteine is added for conjugation. The peptides can be used as tandems and tandem dimers

The present invention also discloses the use of an immunogenic protein and/or part thereof and/or an immunogenic peptide, selectable with a method as described above for the preparation of a medicament.

In another embodiment of the invention, specific emerging B cells can be sorted and isolated by for example cell sorting and/or magnetic bead separation.

Said isolated B cells can be immortalized according to methods generally known in the field of hybridoma production and thus become immortalized B cells producing monoclonal antibodies. Therefore, the present invention also discloses a B-cell selected with the method as described above for the preparation of an antibody, and the use of such a B cell for the production of a hybridoma.

Monoclonal antibodies produced by said B cell hybridoma are particularly useful for diagnostic purposes or for the preparation of a medicament, for example for the inactivation of GnRH in the circulation or for the specific destruction of a tumour cell, or for inhibiting the replication of a pathogen. Therefore, the present invention also discloses an isolated antibody produced by above-described B cell and the use of said antibody for the preparation of a medicament

The techniques described in this patent application may be used for selecting suitable antigens for inducing an immune response. Once an antigen is known, the present application shows how to increase the labelling sensitivity by making binding molecules comprising multiple copies of said antigen and how to label the binding molecules and how to use the labelled binding molecules for the selection of an antigen. Therefore, this application discloses a kit of parts comprising at least a first binding molecule associated with a first label and a second binding molecule associated with a second label, said binding molecules comprising at least two binding regions for the same target molecule. A kit as above described is very useful to select a peptide from a library of peptides. Therefore, the present invention discloses the use of said kit of for the selection of an antigen.

The invention is further explained in the examples, without being restricted to it.

### Example 1

### Detection of low-frequency early B cells.

### Materials and Methods

### Animals

6-8 week old mice were used and were treated according to the ethical guidelines of our institutions. Mice were immunized intraperitoneally on day 0 with GnRH-TDK coupled with ovalbumin (100 µg) or with Gastrin-TDK1 coupled with diphtheria toxoid. (Pepscan Systems, Lelystad, NL), in Complete Freunds Adjuvant. Control mice were not immunized. On day 7, half of the mice of each group were boosted, using Incomplete Freunds Adjuvant, and half of the animals were sacrificed and spleens were harvested. On day 14, spleens were harvested from the remaining mice. All animal experiments were done with permission from the local ethical committee (DEC).

### Tetramer synthesis

Tetrameric molecules were produced by mixing equal volumes of C-biotinylated, middle biotinylated or N-biotinylated GnRH at 200 µM (Pepscan Systems) with R-phycoerythrin (PE)-labelled neutravidin at 3.3 µM (PE-NA, Molecular Probes, Eugene, OR), or allophycocyanin (APC)-labelled streptavidin at 6.1 µM (APC-SA, Molecular Probes). Each mixture was incubated at 4°C for at least 12 hours. Then, tetramers were separated from non-complexed peptide by gel filtration using Bio-Gel P-30 spin columns (Bio-Rad, Hercules, CA).

The following biotinylated gastrin peptide was used to form a tetramer: pEGPWLEEEEEAYGWMDFK($)# , wherein # = amide; pE = pyroGlutamine and K($) = biotinylated Lysine

Each tetramer was titrated in order to determine the optimal concentration.

### Flow cytometry

Cell suspensions of splenocytes were obtained by mechanical disruption of the spleens, and erythrocytes were lysed in 0.16 M chloride ammonium solution pH 7.4. The resulting single cell suspensions were stained with combinations of fluorochrome-labelled antibodies and GnRH tetramers at optimal dilution. Staining was conducted at 4°C in two steps. First, cells were stained with the PE-labelled tetramer for one hour, using the empirically determined optimal concentration. Cells were then washed three times with FACS buffer. Next, cells were stained with the APC- or FITC-labelled tetramer and combinations of PerCP-labelled anti-CD3 antibody (clone 145-2C11, BD Biosciences), FITC-labelled anti-CD19 antibody (clone 1D3, BD Biosciences), PE-labelled anti-CD27 antibody (clone LG.3A10, BD Biosciences) and PE-labelled CD138 antibody (clone 281-2, BD Biosciences). For the staining experiments involving directly labelled ligands, we used FITC- and biotin-labelled GnRH (Pepscan Systems).

Data were acquired using a FACSCalibur flow cytometer and CellQuest software (BD Immunocytometry Systems). 1.106 events were acquired per sample. For FACS analysis lymphocytes were positively gated based on their forward and side scatter profile (live lymphocyte gate), and irrelevant T cells and auto-fluorescent cells were excluded with a negative gating on CD3-PerCP.

**ELIspot assay.** Nitrocellulose bottomed 96-well Multiscreen HA filtration plates (Millipore) were coated with 20 µg/ml of antigen or goat anti-mouse IgG antibody (SBA, Cat. No 1031-01) and incubated at 4°C overnight. Wells were washed 3 times with PBS and blocked for 30 minutes with PBS 5% FCS. Blocking medium was replaced with 100 µl of RMPI medium 10% FCS and the resuspended cells from the cell cultures. Plates were incubated for 24 h at 37°C in incubator 6% CO₂. Wells were washed 3 times with PBS 0.05% Tween-20. 100 µl/well of AP-conjugated goat anti-mouse IgG (SBA, Cat. No 1030-04) was added and incubates for 2-4 h at room temperature. Wells were washed 3 times with PBA 0.05% Tween-20. 100 µl/well of BCIP/NBT substrate were added. After spots development the plates were washed in tap water. The number of the spot was evaluated using A.EL.VIS software.

**Elisa test.** 96-well multi-well plates were coated with 20 µg/ml of antigen or goat anti-mouse IgG antibody (SBA, Cat. No 1031-01) and incubated at 4°C overnight. Wells were washed 3 times with PBS 0.05% Tween-20 and blocked for 30 minutes with PBS 5% BSA (Sigma). Wells were washed 3 times with PBS 0.05% Tween-20. In each well the samples were added and incubated for 1h.Wells were washed 3 times with PBS 0.05% Tween-20. 100 µl/well of PO-conjugated goat anti-mouse IgG was added and incubated for 1 h at room temperature. Wells were washed 3 times with PBA 0.05% Tween-20. 100 µl/well of ASBT substrate were added. After 3 minutes of incubation the plates were read with an Elisa reader (Bio Rad).

### Anti-peptide serum antibody level: radio-autoimmune assay.

Antibody titres against GnRH were determined with a radio-immune assay (RIA) as described by Meloen et al. (1994). An amount of 50 µl antiserum diluted 1:1000 in PBS with 0.4% bovine serum albumin (BSA) was allowed to bind with iodinated GnRH (Amersham Pharmacia Biotech, Buckinghamshire, England) in 50 µl PBS with 0.4% BSA. After incubation for two days at 4°C, the iodinated GnRH bound to the antibodies was separated from the unbound using dextran-coated charcoal. The supernatant was counted and the percentage of iodinated GnRH bound by the antibodies in the 1:2000 diluted serum was calculated.

### Results

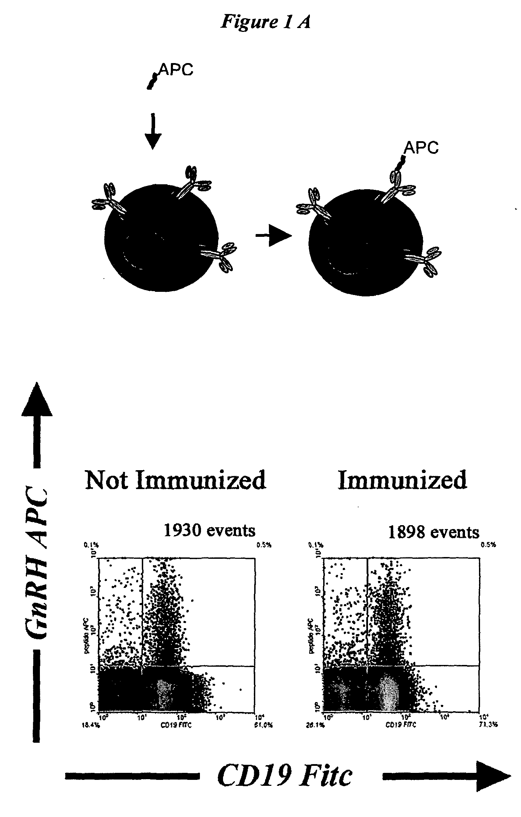

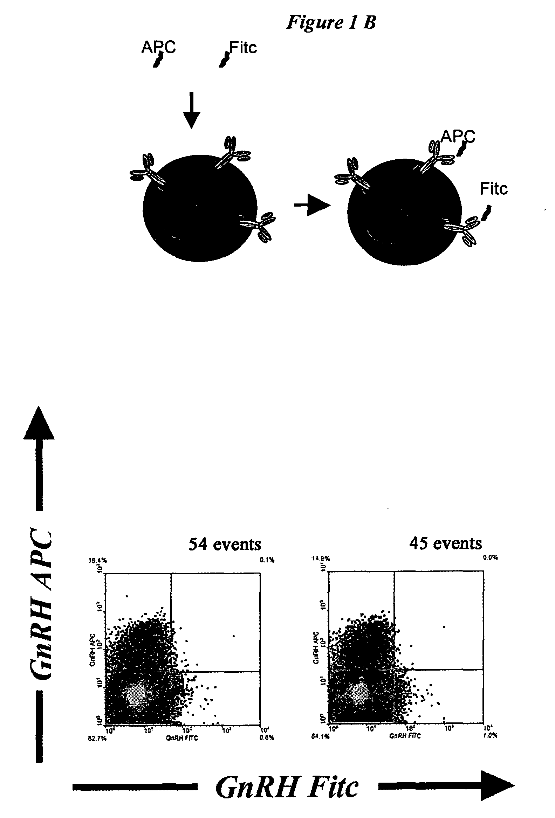

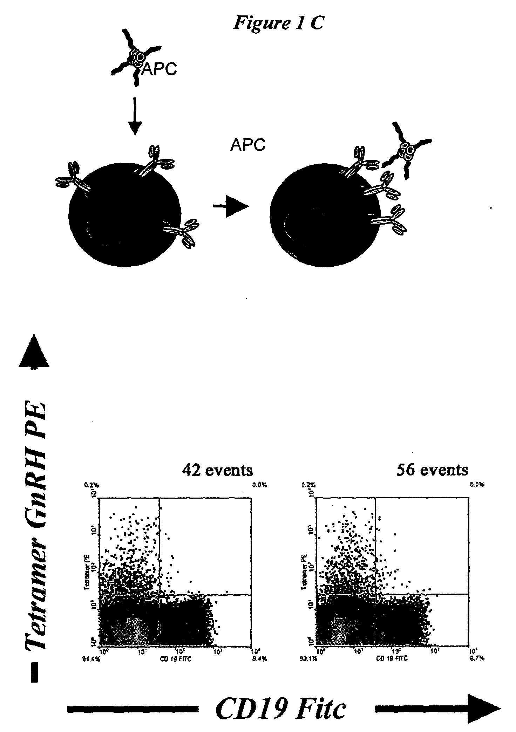

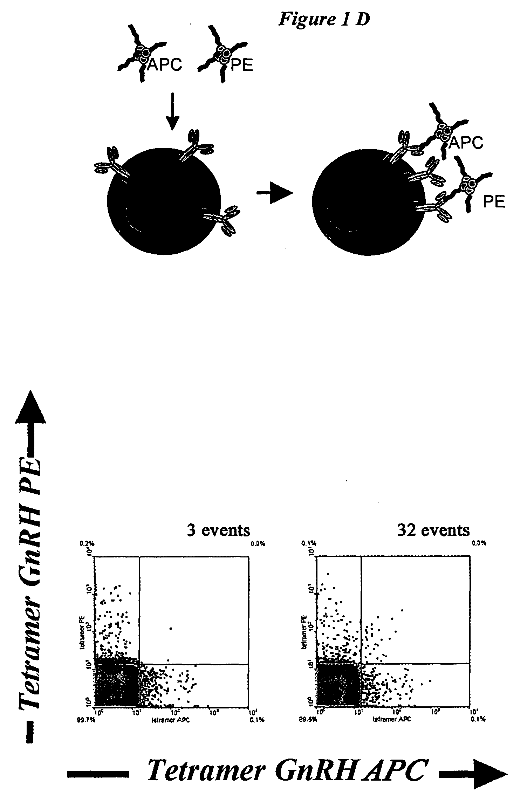

We developed a novel B cell detection strategy able to lower the discrimination's level to 20-30 specific cells in one million of lymphocytes using tetrameric molecules. This method employed two different fluorescent-labelled molecules for each peptide, allowing improved signal to noise ratios and consequently, a very high sensitivity. To demonstrate the utility of our approach to detect ultra low frequency B cells, we used as two model vaccine formulations: GnRH-like peptide and Gastrin-like peptide. Several methods have been described to detect antigen-specific B cells using flow cytometry (McHeyzer-Williams *et al., 1**993***; Townsend *et al.,* 2001; Newman *et al.,* 2003), as shown schematically in Fig.1.

To detect GnRH-specific B cells we used directly labelled native GnRH, or labelled tetramers with native GnRH, both in single and double staining protocols. GnRH tetramers were produced by incubating biotinylated GnRH with fluorescent-labelled streptavidin or neutravidin. Monoclonal antibodies against the B-cell marker CD19 and the T-cell marker CD3 were included in the staining procedure. The anti-CD3 staining was used to negatively select (irrelevant) T cells, as well as auto-fluorescent cells. As shown in Fig.1, our results demonstrated that only double tetramer staining produced discriminating results (Fig. 1D), and none of the other staining methods (Fig.1 A-C). Whereas direct staining with labelled GnRH (Fig. 1 A,B) or with a single tetramer according to the method of Newman *et al*. (Fig.1 C) produced only high background signals, and very little specific signal in the case of double staining with labelled GnRH according to Townsend *et al* (Fig. 1 B). We found that the double tetramer staining as disclosed in this application detects specific B cells at a frequency of 20-30 per 10⁶ splenocytes at 7 days after a single immunization (Fig.1 D).

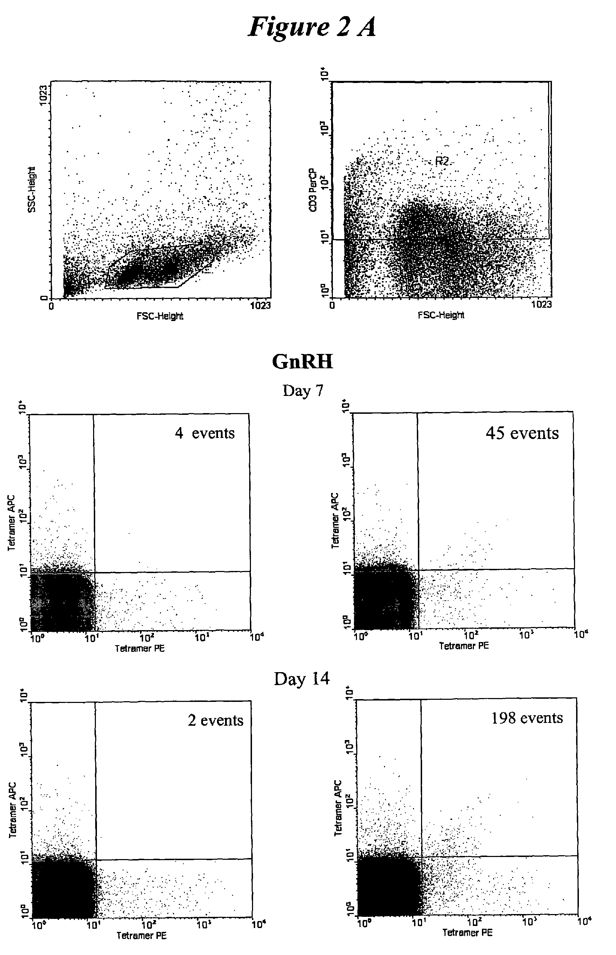

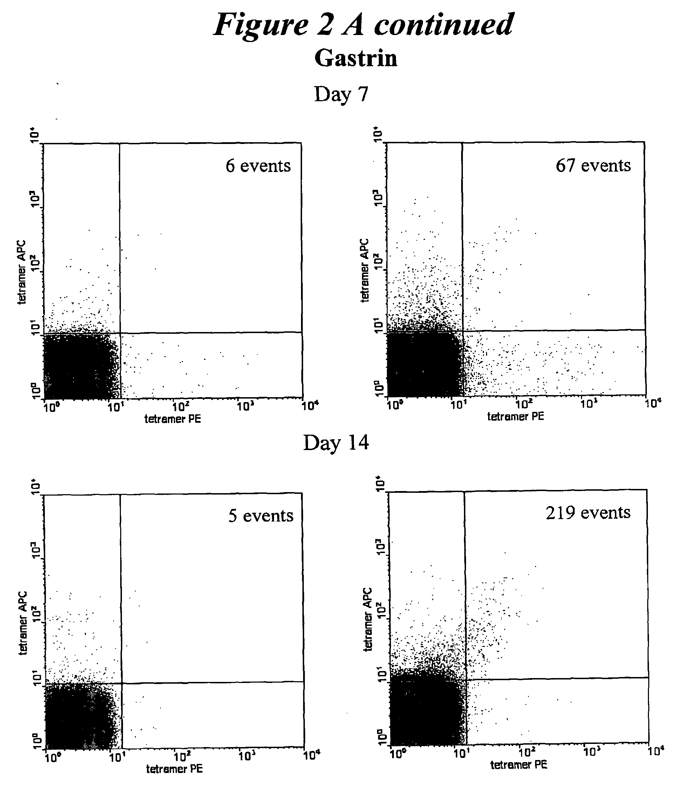

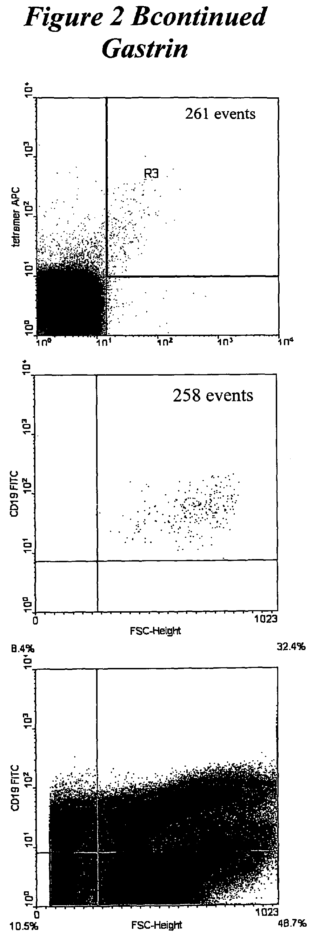

Lymphocytes were stained with peptide-tetramers, and monoclonal antibody against CD19 and CD3. Since we expected only a very small population of specific B cells, debris were excluded with a live gate (forward and side scatter), and T cells were excluded with a negative gating on CD3e. Biotinylated GnRH tetrameric molecules or biotinylated gastrin tetrameric molecules were used in a double staining strategy to identify peptide-specific cells (Fig 2A). Using this approach, we were able to detect 20-30 specific peptide-tetramers double-positive cells in one million lymphocytes at day 7 after immunization, and 100 specific cells at day 14 after immunization (Fig. 2A).

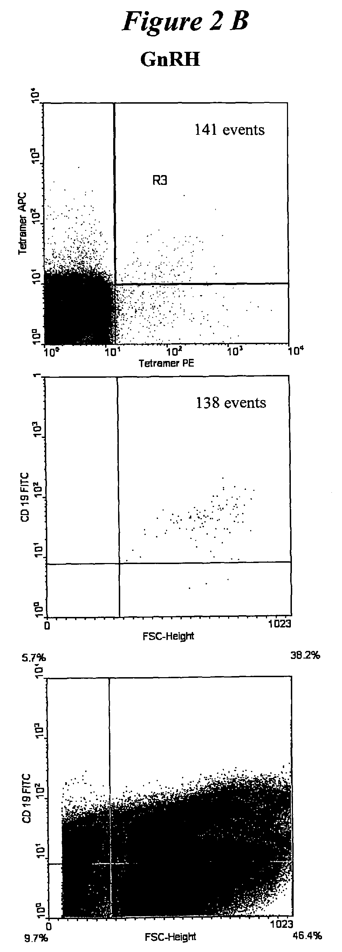

After the discrimination of the peptide-tetramer double-positive cells, we studied the phenotypic characteristic of these cells. Although several authors have used different markers for the characterization of mature B cells, we decided to use CD19 as a pan B-cell marker (McHeyzer -Williams *et al.,* 1993). All the double-positive cells were CD19 positive classifying this cells as B cells. Interestingly, the rare double-positive cells found in the non-immunized mice are also CD19 positive (Fig. 2B) showing that these cells are the B cell precursor population for the specific antigen that comprises approximately 3-4 cells per million of lymphocytes in naive mice.

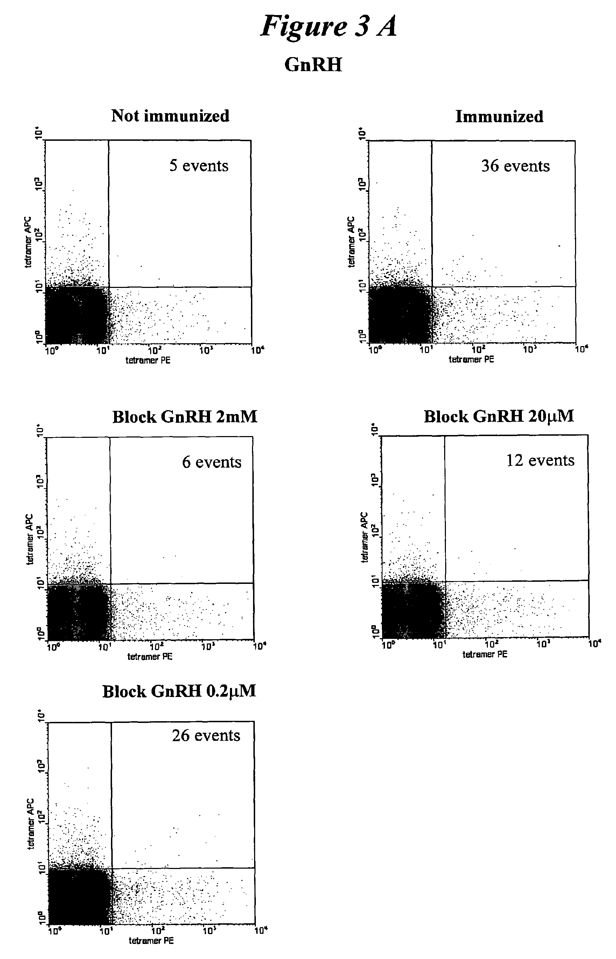

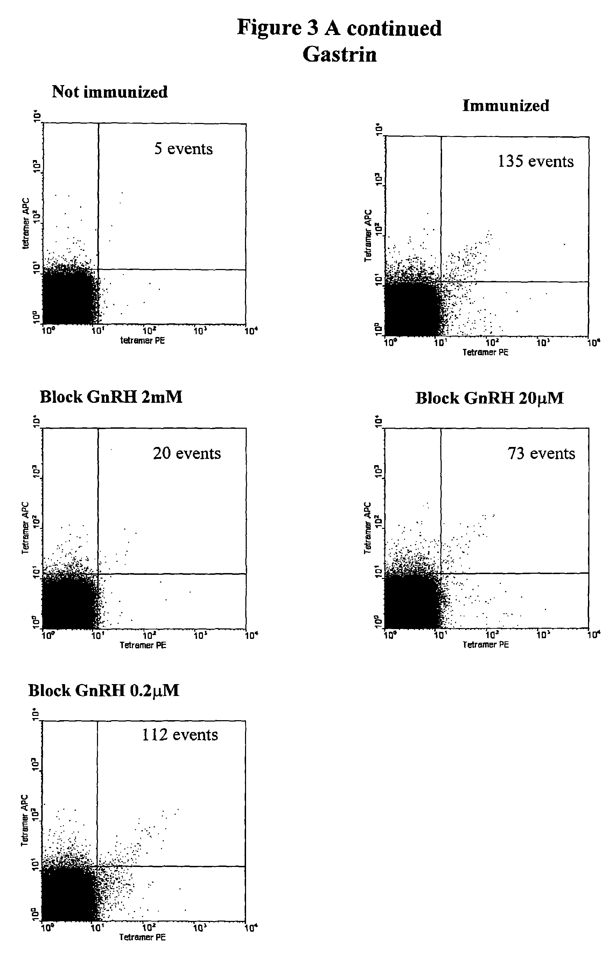

The specificity of tetramer binding was demonstrated by inhibition studies with unlabeled GnRH (see figure 3A) or Gastrin (data not shown). Lymphocytes were preincubated with unlabeled peptide followed by incubation with labelled tetramers, CD19 and CD3e antibodies. As shown in Fig 3A tetramer staining was inhibited by inclusion of unlabeled peptide in a dose-dependent manner.

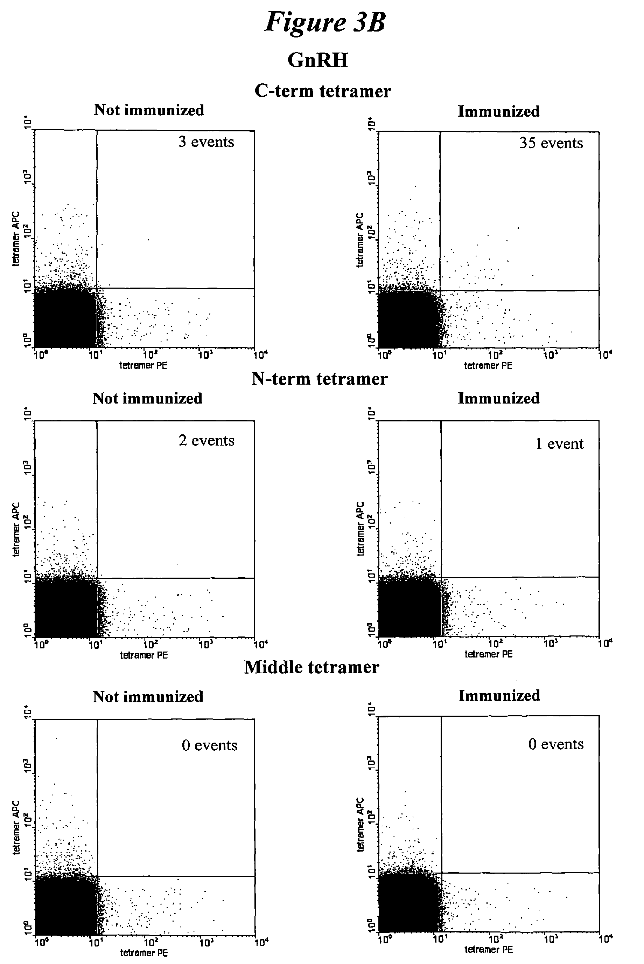

As a further specificity test, we produced tetramers using peptides that were N-, middle, or C-terminally labelled. As shown in fig 3B, we found that only the C-term biotinylated GnRH tetrameric were able to detect the specific B cells.

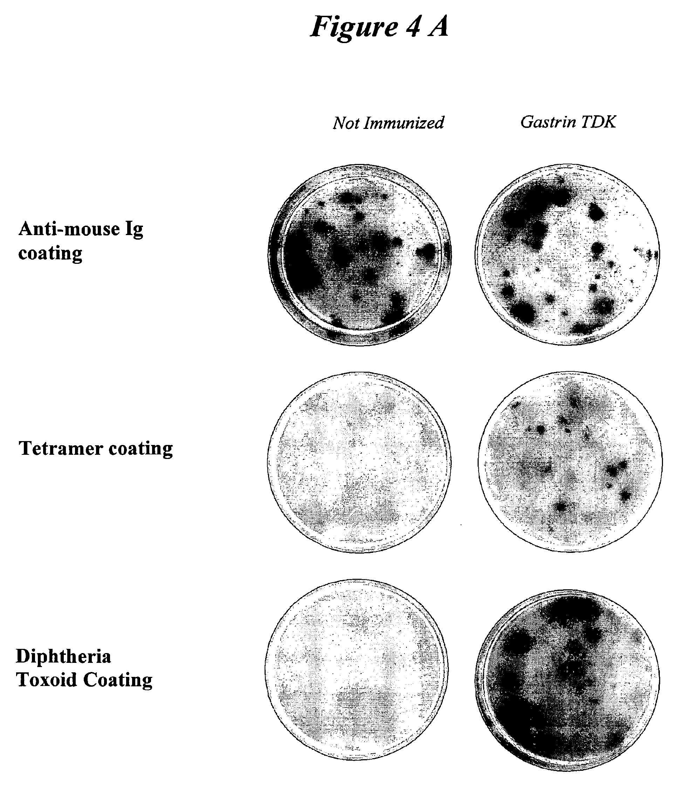

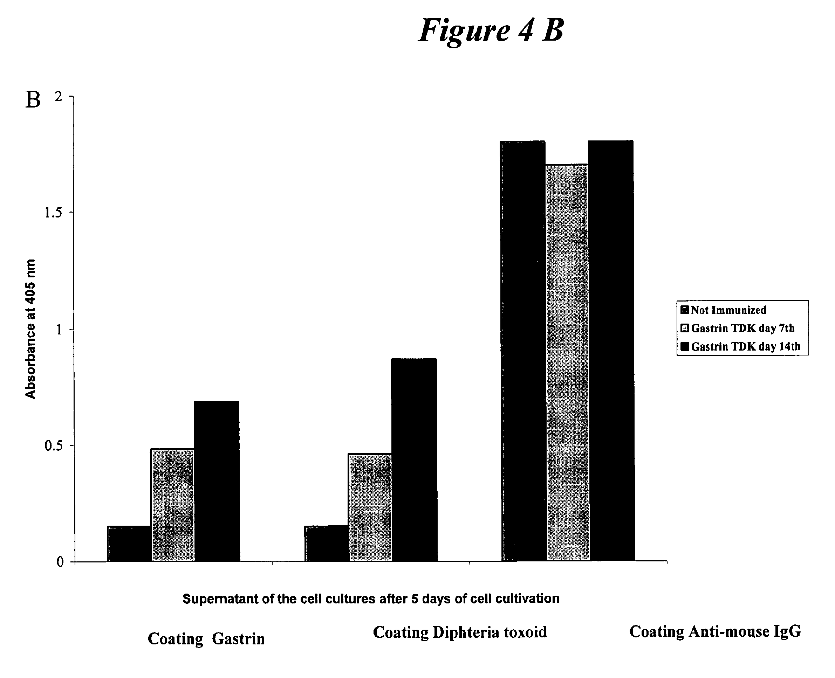

After removing the erythrocytes from the spleen samples, splenocytes from gastrin-immunized mice were cultivated for 5 days under IL-2 and Pansorbin stimulation. At the end of this time the supernatants were used for performing an ELISA test, and the cells were used in an ELIspot assay. As shown in fig.4A, only the cells of the immunized mice were able to produce spots on the ELIspot well. These results were confirmed by ELISA test (Fig 4B) and they are consistent with the results of FACS analysis, proving that B cell detection is a reliable method for selecting peptide vaccines.

Therefore, in the present study, we have disclosed a novel method to visualize antigen-specific B cells at very low frequencies. In our experiments, the double tetramer staining procedure was more sensitive and more reproducible then the previously described methods (e.g., labelled peptide ligands, single tetramers). Our peptide tetramers have proven to be highly specific B cell staining reagents.

### Example 2

### Comparison of the immunogenicity of GnRH derived peptides.

### Materials and Methods

### Animals

6-8 week old mice were used and treated for according to the ethical guidelines of University of Utrecht. Mice were immunized intraperitoneally on day 0 with , GnRH-like peptides conjugated to ovalbumin (OVA). We prepared three different peptides, i.e., GnRH-monomer (pEHWSYGLRPGC), GnRH-tandem (pEHWSYGLRPGQHWSYGLRPGC) and GnRH tandem dimer (TDK, the dimerized form of pEHWSYkLRPGQHWSYkLRPGC in which 'k' represents a D-lysine residue) (Pepscan Systems, Lelystad, NL), in Complete Freunds Adjuvant. Control mice were not immunized. On day 7 half of the mice of each group were boosted, using Incomplete Freunds Adjuvant, and half of the animals were sacrificed and spleens were harvested. On day 14, spleens were harvested from the remaining mice. All animal experiments were done with permission from the local ethical committee (DEC).

**Cell cultivation.** Cell suspensions of splenocytes were cleared of erythrocytes and the cells were cultured in T75 flasks in the presence of Pansorbin (SAC, Calbiochem, Cat. No 507858) and recombinant human IL-2 (CETUS) for 5 days in incubator at 37 °C and 5% CO₂. Cells were cultivated 5-10.10⁷ spleen cells at 2.10⁶/ml in RMPI 10% FCS. After the incubation the cells were collected in tubes and centrifuged at 1200 rpm 10'. Supernatants were used for Elisa tests.

**Tetramer synthesis.** Tetrameric molecules were produced by mixing an equal amount of C-term biotinylated peptide at 200 µM (Pepscan B.V.) with R-phycoerythrin (PE)-labelled neutravidin at 3.3 µM (PE-NA, Molecular Probes, Eugene, OR), or allophycocyanin (APC)-labelled streptavidin at 6.1 µM (APC-SA, Molecular Probes). Each mixture was incubated at 4°C for a minimum of 12 hours. Than, tetramers were separated from non-complexed peptide by gel filtration using a Bio-Gel P-30 spin column (Bio-Rad, Hercules, CA). The amount of tetramers to use was determined by titration experiments. Peptides used were derived from GnRH (Pepscan B.V.).

**Flow cytometry.** Cell suspension of splenocytes was obtained by mechanical disruption of the spleens, and erythrocytes were lysed in 0.16 M chloride ammonium solution pH 7.4. The resultant cell suspensions were stained with combinations of fluorochrome-labelled antibodies and peptide's tetrameric molecules at optimal dilution. Staining was routinely conducted at 4°C in two steps: first with peptide's tetrameric molecule labelled with PE for one hour. Cells were then washed three times with FACS Buffer. Next, cells were stained with the APC- or FITC-labelled tetramer and combinations of PerCP-labelled anti-CD3 antibody (clone 145-2C11, BD Biosciences), and FITC-labelled anti-CD19 antibody (clone 1D3, BD Biosciences) were used for the second step. Data were acquired using a FACSCalibur flow cytometer and Cell Quest software (BD Immunocytometry Systems). 1.10⁶ events were acquired per sample. All data are representative plots derived from a minimum of three independent experiments in which at least three experimental and three control mice were analysed.

### Results

To detect GnRH-specific B cells we used labelled tetramers with native GnRH, in double staining protocols. Monoclonal antibodies against the B-cell marker CD19 and the T-cell marker CD3 were included in the staining procedure. The anti-CD3 staining was used to negatively select (irrelevant) T cells, as well as auto-fluorescent cells.

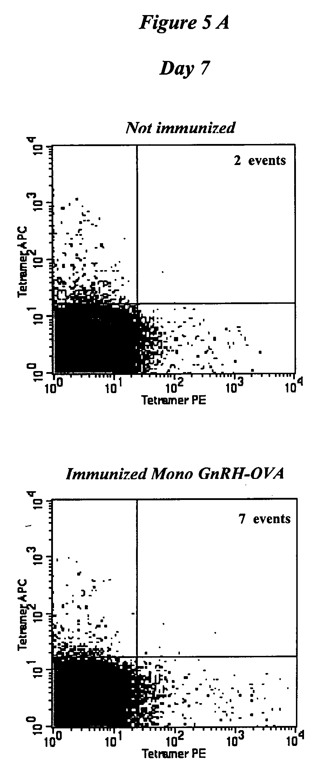

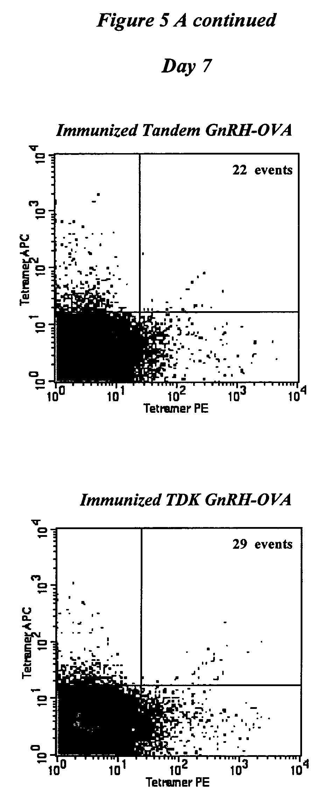

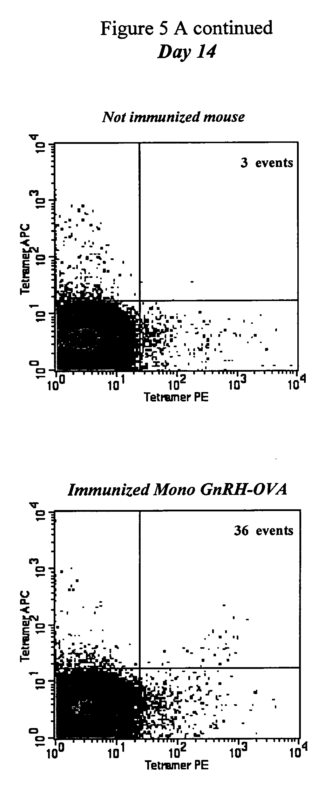

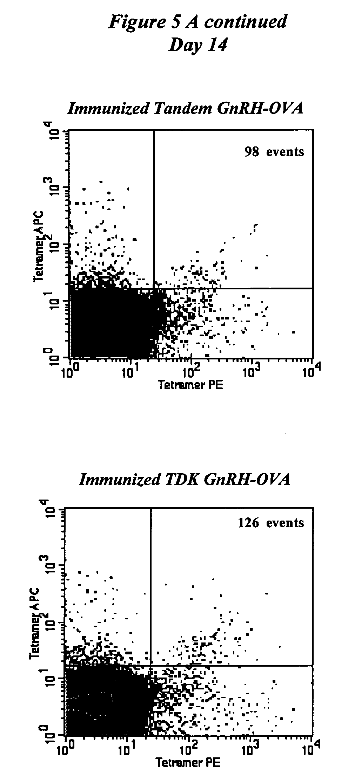

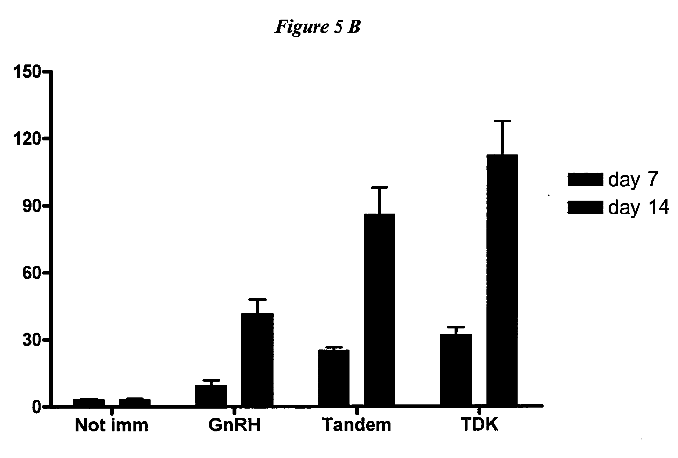

With the above-described method we compared the three different peptide formulations of GnRH to vaccinate the mice. The results of the double tetramer staining assay are given in Figure 5 A and 5 B. The results clearly show that GnRH TDK-OVA caused the most extensive B cell expansion, which was measured at both 7 and 14 days after vaccination.

### Example 3

### Comparison of the immunogenicity of Gastrin derived peptides.

With the above-described method we compared four different peptide formulations of Gastrin to vaccinate the mice.

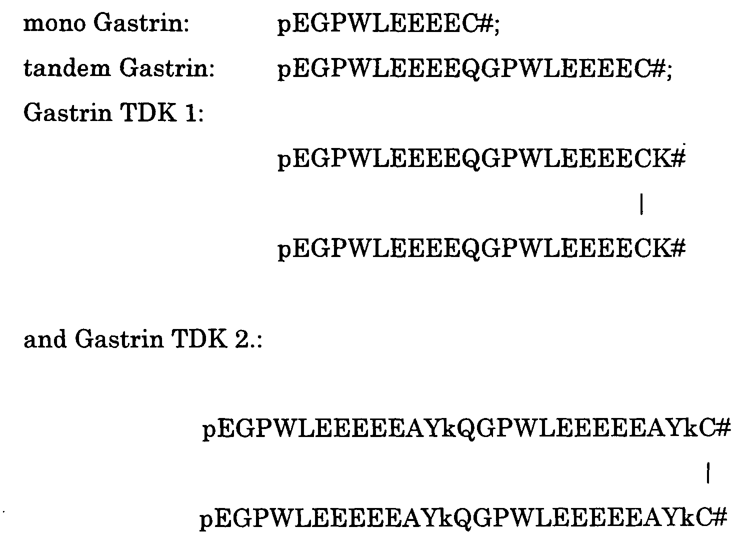

These peptides were tested as a vaccine in mice and at 7 and 14 days after immunization specific B cells were detected with the method of the invention using tetramer Gastrin peptide. The following biotinylated gastrin peptide was used to form a tetramer: pEGPWLEEEEEAYGWMDFK($)# , wherein # = amide; pE = pyroGlutamine and K($) = biotinylated Lysine

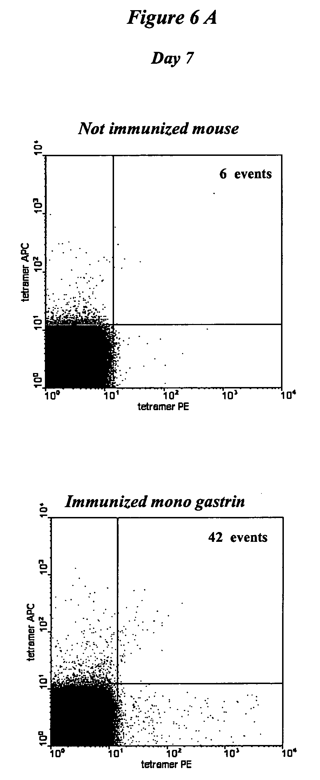

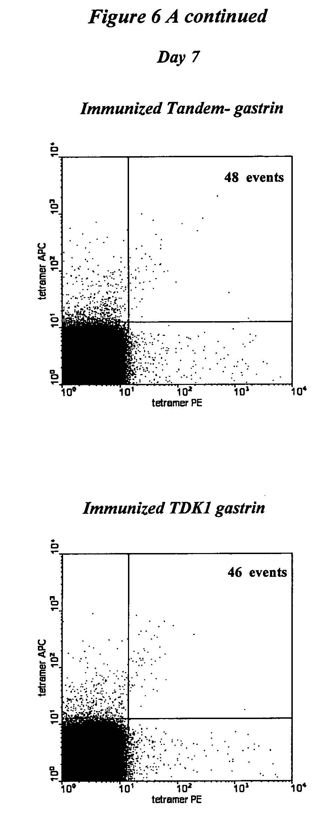

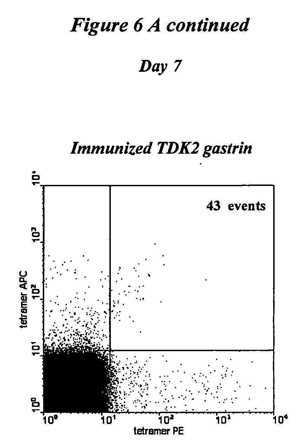

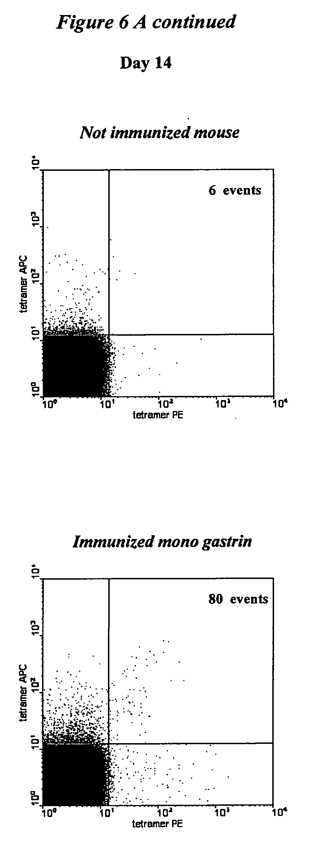

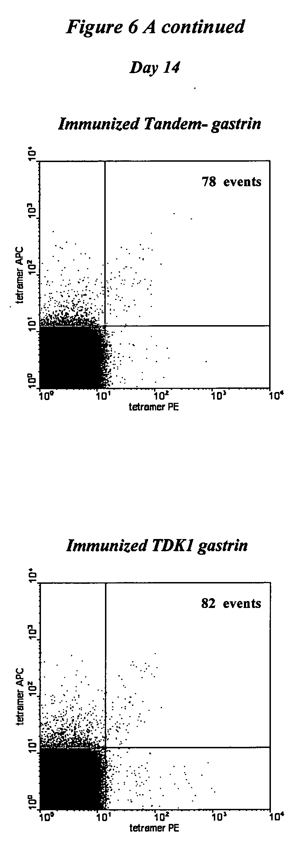

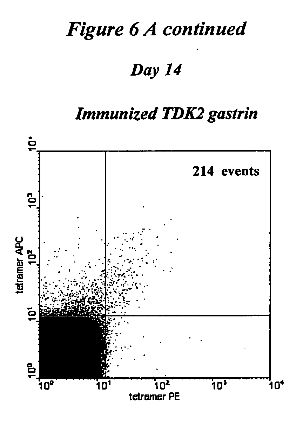

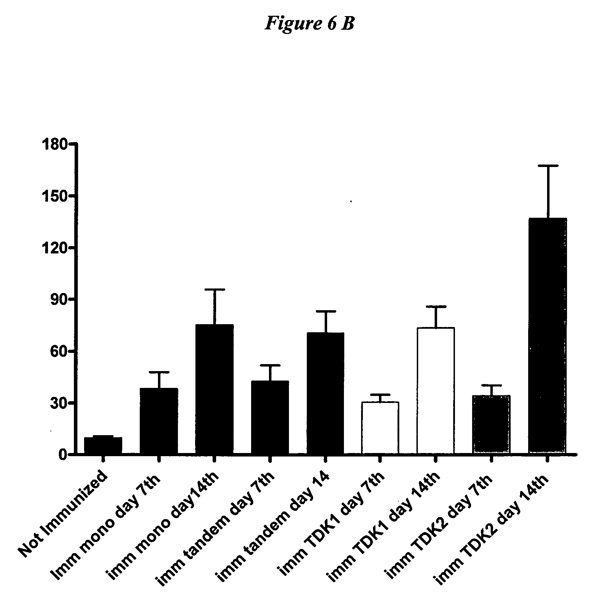

The results of the double tetramer staining assay are presented in Figure 6 A and 6 B. The results clearly show that Gastrin TDK2 -OVA caused the most extensive B cell expansion, which was measured at 14 days after vaccination.

### Legends to the figures

**Figure 1-** *Visualization of antigen-specific B cells.* Several flow cytometry strategies have been developed to detect specific B cells, and we have compared their effectiveness.
(A) Staining with a single fluorescent labelled peptide. Splenocytes from immunized and not immunized mice stained with C-biotinylated GnRH and FITC-labelled anti-CD19, followed by staining with APC-labelled streptavidin.
(B) Splenocytes from immunized (I) and not immunized mice (N) were stained with C-biotinylated and FITC-labelled GnRH, followed by staining with APC-labelled streptavidin (according to Townsend *et al*.2001)
(C) Staining with a single fluorescent labelled tetramer (according to Newman *et al.,* 2003). Splenocytes from immunized (I) and not immunized mice (N) were stained with the PE-labelled GnRH tetramer and FITC-labelled anti-CD19.
(D) Staining with 2 fluorescent labelled tetramers, using the single epitope multiple staining' principle of this application. Splenocytes from immunized (I) and not immunized mice (N) were stained with the PE-labelled and APC-labelled GnRH tetramers and with FITC-labelled anti-CD19.

**Figure 2-** *Identification of GnRH and of Gastrin specific B cells.*
A) Specific B cell tetramer double staining. Splenocytes from mice immunized with GnRH-TDK or Gastrin-TDK were stained with peptide-tetramer molecules, anti-mouse CD3e, anti-mouse CD19 and analysed by FACS. Gating strategy for identification of tetramer-reactive lymphocytes includes a live gate (forward and side scatter) and a negative gating on CD3e for excluding T cells. Using two different peptide-tetramer molecules, linked with PE or APC, we were able to detect specific GnRH cells at 7 and 14 days after immunization. All Dot Plots derived from a minimum of three independent experiments in which at least 5 experimental and 5 control mice were analysed.
B) Specific tetramer double positive cells are CD 19 positive. After the identification of the tetramer double positive cells, we made a gate (R3) to visualize only these cells. Then, we looked at the phenotypic expression of CD19 on these cells. All the specific cells are CD19 positive. The density plots representative of all the lymphocytes population and they were made to select the right size of the quadrants for the discrimination of the CD 19 positive cells.

**Figure 3-** *Specificity of the B cell detection.*
A) Not labelled GnRH or Gastrin is able to inhibit the peptide-tetrameric molecules staining of the specific B cells. The specificity of tetramer binding was demonstrated by inhibition studies. Splenocytes were pre-incubated with unlabeled GnRH or Gastrin, followed by incubation with labelled tetramers and anti-CD19 and anti-CD3 antibodies. Three doses of unlabelled antigens were used: 2 mM, 20 µM and 0.2 µM. Tetramer staining was nearly abolished by the higher dose.
B) Specific B cells are recognized only by C-term tetramer. Peptide-tetrameric molecules made with different biotinylated GnRH: C-term-, N-term-, and middle biotinylated peptides. Only the C-term peptide was able to detect the specific cells.

**Figure 4-** *Detection of antigen specific B cells by ELIspot assay and Elisa test*.
A) ELIspot assay. After lysing the erythrocytes, splenocytes, from not immunized mice and from mice 14 days after immunization, were cultivated for 5 days under Pansorbin and IL-2 stimulation. After this incubation, cells were added to ELIspot wells coated with peptide-tetramer, diphtheria toxoid and anti-mouse IgG. On the wells coated with anti-mouse IgG it is possible to evaluate the total B cell response of the spleens. On the others it is possible to evaluate the specific B cell response against the antigen. Only cells from immunized mice were able to produce spots on the specific antigen.
B) Elisa test. The supernatants collected from the cells after 5 days of culture are used to perform an Elisa test. The level of total B cell response is identical between immunized and not immunized mice. Only sera from immunized mice contain antibodies against gastrin and diphtheria toxoid. The levels of specific antibodies are higher 14 days after immunization than after 7. These results are consistent with the FACS analysis.

**Figure 5-** *Counting GnRH specific B cells after immunization with the three GnRH vaccines*.
(A) Specific B cell tetramer double staining. Splenocytes from mice immunized with GnRH-mono, GnRH tandem-, and TDK tandem dimer were stained with GnRH-tetramers (PE and APC), PerCP labelled anti-CD3, FITC labelled anti-CD19 and analysed by flow cytometry. Tetramer-binding lymphocytes were identified using a live gate (forward and side scatter) and a negative gate on FL3+ to exclude (irrelevant) T cells, and autofluorescent cells. For each density plot, 1x10⁶ events were acquired. Shown in the upper right panel are the double positive-specific B cells. Plots shown are representative from a minimum of three independent experiments in which at least 3 experimental and 3 control mice were analysed.
(B) For each vaccine, at least 9 samples were analysed 7 days and 14 days after immunization. Shown in the graph is the mean of the number of double positive cells found in each group.

**Figure 6-***Counting Gastrin specific B cells after immunization with the four Gastrin vaccines.*
A Specific B cell tetramer double staining. Splenocytes from mice immunized with Gastrin-mono, Gastrin tandem-, TDK1 tandem dimer and TDK2 tandem dimer were stained with Gastrin-tetramers (PE and APC), PerCP labelled anti-CD3, FITC labelled anti-CD19 and analysed by flow cytometry. Tetramer-binding lymphocytes were identified using a live gate (forward and side scatter) and a negative gate on FL3+ to exclude (irrelevant) T cells, and autofluorescent cells. For each density plot, 1x10⁶ events were acquired. Shown in the upper right panel are the double positive-specific B cells. Plots shown are representative from a minimum of three independent experiments in which at least 3 experimental and 3 control mice were analysed.
(B) For each vaccine, at least 9 samples were analysed 7 days and 14 days after immunization. Shown in the graph is the mean of the number of double positive cells found in each group.

### References

Adams, C.L., MacLeod, M.K.L., Milner-White, E.J., Aitken, R., Garside, P. and Stott, D.I. Complete analysis of the B cell response to a protein antigen, from in vivo germinal centre formation to 3D modelling affinity maturation. *Immunology* **108**, 274-287 (2003).

Altman, J.D., Moss, P.A.H., Goulder, P.J.R., Barouch, D.H., McHeyzer-Williams, M., Bell, J., McMichael, A.J., Davis, M.M. Phenotypic analysis of antigen-specific lymphocytes. *Science* **274**, 94-96 (1996).

Bateman, H.E., B.S. Kasimis, C.R. Yook and V. Hiremath. 1995. Case report: primary choriocarcinoma of the stomach. N J Med 92:459-462.

Carpelan-Holmstrom, M., C. Haglund, J. Lundin, H. Alfthan, U.H. Stenman and P.J. Roberts. 1996. Independent prognostic value of preoperative serum markers CA 242, specific tissue polypeptide antigen and human chorionic gonadotrophin beta, but not of carcinoembryonic antigen or tissue polypeptide antigen in colorectal cancer. Br J Cancer 74:925-929.

Cauchi, M.N., S.H. Koh, D. Lim and D.L. Hay. 1981. Oncofoetal antigens in cancer of the cervix and ovary. Br J Cancer 44:403-409.

Davis, M.M., Bjorkman, P.J., T-cell antigen receptor genes and T-cell recognition. Nature 334, 395 (1988).

Fuerst, J., Fiebiger, E., Jungwirth, A., Mack, D., Talwar, P.G., Frick, J., Rovan, E. Effect of active immunization against LHRH on androgen sensitive Dunning R3327-PAP and androgen independent Dunning R3327-AT2.1 prostate cancer cell lines. *Prostate* **32,** 77-84 (1997).

Fujimaki, T., K. Mishima, A. Asai, K. Tabuchi, M. Kobayashi, I. Suzuki and T. Kirino. 2000. Levels of beta-human chorionic gonadotropin in cerebrospinal fluid of patients with malignant germ cell tumor can be used to detect early recurrence and monitor the response to treatment. Jpn J Clin Oncol 30:291-294.

Hara, I., Y. Yamada, H. Miyake, S. Hara, A. Gotoh, M. Fujisawa, H. Okada, S. Arakawa and S. Kamidono. 2002. Detection of beta-human chorionic gonadotropin expressing cells by nested reverse transcriptase-polymerase chain reaction in the peripheral blood stem cells of patients with advanced germ cell tumor. J Urol 167:1487-1491.

Higashida, T., T. Koizumi, S. Yamaguchi, T. Ichimura, K. Hasegawa and R. Nishimura. 2001. Ovarian malignant mixed mesodermal tumor producing the free form of the beta-subunit of human chorionic gonadotropin. Int J Clin Oncol 6:97-100.

Hoermann, R., A.L. Gerbes, G. Spoettl, D. Jungst and K. Mann. 1992. Immunoreactive human chorionic gonadotropin and its free beta subunit in serum and ascites of patients with malignant tumors. Cancer Res 52:1520-1524.

Hoosein, N.M., Kiener, P.A., Curry, R.C., Brattain, M.G. Evidence for autocrine growth stimulation of cultured colon tumor cells by a Gastrin cholecystokinin like peptide. *Exp. Cel. Res.* **186**, 15-21 (1990).

Hotakainen, K., C. Haglund, A. Paju, S. Nordling, H. Alfthan, E. Rintala and U.H. Stenman. 2002. Chorionic Gonadotropin beta-Subunit and Core Fragment in Bladder Cancer: mRNA and Protein Expression in Urine, Serum and Tissue. Eur Urol 41:677-685.

Hotakainen, K., B. Ljungberg, A. Paju, T. Rasmuson, H. Alfthan and U.H. Stenman. 2002. The free beta-subunit of human chorionic gonadotropin as a prognostic factor in renal cell carcinoma. Br J Cancer 86:185-189.

Jackson, D.C., Purcell, A.W., Fitzmaurice, C.J., Zeng, W., and Hart D.N.J. The central role played by peptides in the immune response and the design of peptide-based vaccines against infectious diseases and cancer. *Current Drug Targets* **3**, 175-196 (2002).

Jensen, R.T. Involvement of cholecystokinin Gastrin related peptides and their receptors in clinical gastrointestinal disorders. *Pharmacology and Toxicology* **91**, 333-350 (2002).

Koh, T.J., Dockray, G.J., Varro, A., Cahill, R.J., Dangler, C.A., Fox, J.G., Wang, T.C., Overexpression of glycine extended Gastrin in transgenic mice results in increased colonic proliferation. *J. Clin. Invest.* **103,** 1119-1126 (1999).

Kouskoff, V., Lacaud, G., Pape, K., Retter, M., Nemazee, D., B cell receptor expression level determines the fate of developing B lymphocytes: receptor editing versus selection. *PNAS*. **13**, 743507439 (2000)

Liu, E., Abiru, N., Moriyama, H., Miao, D., Eisenbarth, G.S. Induction of insulin auto-antibodies and protection from diabetes with subcutaneous insulin B:9-23 peptide without adjuvant. *Ann N Y Acad Sci.* **958,** 224-7 (2002).

Lundin, M., S. Nordling, M. Carpelan-Holmstrom, J. Louhimo, H. Alfthan, U.H. Stenman and C. Haglund. 2000. A comparison of serum and tissue hCG beta as prognostic markers in colorectal cancer. Anticancer Res 20:4949-4951.

Manabe, T., M. Adachi and K. Hirao. 1985. Human chorionic gonadotropin in normal, inflammatory, and carcinomatous gastric tissue. Gastroenterology 89:1319-1325.

McHeyzer-Williams, M.G., McLean, M.J., Lalor, P.A., Nossal, G.J.V. Antigen-driven B cell differentiation in vivo. *J Exp Med.* **178(1),** 295-307 (1993).

Meloen, R.H., Langeveld, J.P.M., Schaaper, W.M.M., Slootstra, J.W. Synthetic peptide vaccines: unexpected fulfilment of discarded hope? *Biologicals* **29**, 233-236 (2001).

Monzavi-Karbassi, B., Cunto-Amesty, G., Luo, P., Kieber-Emmons, T. Peptide mimotopes as surrogate antigens of carbohydrates in vaccine discovery. *Trends Biotechnol.* **20(5)**, 207-14 (2002).

Meyer, T., L.A. Cole, P.I. Richman, H.D. Mitchell, J. Myers and G.J. Rustin. 2001. High levels of hCG in choriocarcinoma can result in renal failure and a false-negative pregnancy test in men. Clin Oncol (R Coll Radiol) 13:301-303.

Newman, J., Rice, J.S., Wang, C., Harris, S.L., Diamond, B. Identification of an antigen specific B cell population. *Journal of Immunological Methods* **272**, 177-187 (2003).

Nicoll, J.A., Wilkinson, D., Holmes, C., Steart, P., Markham, H., Weller, R.O. Neuropathology of human Alzheimer disease after immunization with amyloid-beta peptide: a case report. *Nat. Med.* **9(4)**, 448-52 (2003).

Noguchi, M., Kobayashi, K., Suetsugu, N., Tomiyasu, K., Suekane, S., Yamada, A., Itoh, K., Noda, S. Induction of cellular and humoral immune responses to tumor cells and peptides in HLA-A24 positive hormone-refractory prostate cancer patients by peptide vaccination. *Prostate* **57(1)**, 80-92 (2003).

Oonk, H.B., Turkstra, J.A., Schaaper, W.M.M., Erkens, J.H.F., Schuitemaker de Weerd, M.H., Van Nes, A., Verheijden, J.H.M., and R.H. Meloen. New GnRH like peptide construct to optimize efficient immunocastration of male pigs by immunoneutralization of GnRH. *Vaccine* **16**, 1074-1082 (1998).

Ribas, A., Timmerman, J.M., Butterfield, L.H., Economou, J.S. Determinant spreading and tumor responses after peptide-based cancer immunotherapy. *Trends Immunol.* **24(2),** 58-61 (2003)

Smith, A.M., Justin, T., Michaeli, D., Watson, S. Phase I/II study of G17-DT, an anti-gastrin immunogen, in advanced colorectal cancer. *Clin*. *Canc. Res*. **6,** 4719-4724 (2000)

Stetson, D.B., Mohrs, M., Mallet-Designe, V., Teyton, L., Locksley, R.M. Rapid expansion and IL-4 expression by Leishmania specific naive helper T cells in vivo. *Immunity* **17**, 191-200 (2002).

Syrigos, K.N., I. Fyssas, M.M. Konstandoulakis, K.J. Harrington, S. Papadopoulos, N. Milingos, P. Peveretos and B.C. Golematis. 1998. Beta human chorionic gonadotropin concentrations in serum of patients with pancreatic adenocarcinoma. Gut 42:88-91

Szavay, P.O., C. Wermes, J. Fuchs, M. Schrappe, P. Flemming and D. von Schweinitz. 2000. Effective treatment of infantile choriocarcinoma in the liver with chemotherapy and surgical resection: a case report. J Pediatr Surg 35:1134-1135.

Talwar, G.P. Vaccines and passive immunological approaches for the control of fertility and hormone-dependent cancers. *Immunological review* **171**, 173-192 (1999).

Thorburn, C.M., Friedman, G.D., Dickinson, C.J., Vogelman, J.H., Orentreich, N., Parsonnet, J. Gastrin and colorectal cancer: a prospective study. *Gastroenterology* **115**, 275-280 (1998).

Townsend, S.E., Goodnow, C.C., Cornall, R.J. Single epitope multiple staining to detect ultralow frequency B cells. *Journal of Immunological Methods* **249**, 137-146 (2001).

Uckaya, G., Uckaya, G., A. Ozet, A. Arpaci and S. Komurcu. 1998. Human chorionic gonadotropin and CA 15-3 producing adenocarcinoma. Nuklearmedizin 37:297-298.

Watson, S., Durrant, L. and Morris, D. Gastrin: growth enhancing effects on human gastric and colonic tumor cells. *Br. J. Cancer* **59**, 554-558 (1989).

## Claims

1. An affinity-binding assay comprising a particle having at least four copies of a target molecule and at least two binding molecules specific for said target molecule, wherein a first of said binding molecules is associated with a first label and a second of said binding molecules is associated with a second label, wherein a particle having said first label and a particle having said second label are distinguishable from a particle having both said first and said second label, wherein said first and said second binding molecule each comprise at least two binding regions specific for said target molecule.

2. An affinity-binding assay according to claim 1, wherein said particle comprises a cell.

3. An affinity-binding assay according to claim 2, wherein said cell comprises an activated immune cell in the clonal expansion phase of a primary immune response.

4. An affinity-binding assay according to claim 2, wherein said cell comprises a B cell.

5. An affinity-binding assay according to any of claims 1-4, wherein said particle having said first and said second label increases the sensitivity of the affinity-binding assay.

6. An affinity-binding assay according to any of claims 1-5, wherein said first and said second binding molecule each comprise at least four binding regions specific for said target molecule.

7. An affinity-binding assay according to claim 6, wherein said at least four binding regions are essentially identical.

8. An affinity-binding assay according to any of claims 1-7, wherein said binding molecule represents an epitope of a native protein.

9. An affinity-binding assay according to any of claims 1-8, wherein said target molecule comprises a B cell receptor.

10. An affinity-binding assay according to claims 9, wherein said target molecule comprises a variable region of said a B cell receptor.

11. A composition comprising a first multiple binding molecule associated with a first label and a second multiple binding molecule associated with a second label, wherein the signal obtained from said first label and said second label is distinguishable from the combined signal of said first and second label, wherein each binding molecule comprises at least four binding regions specific for essentially the same target molecule, preferably for essentially the same epitope on said target molecule.

12. A method for selecting a synthetic antigen from a collection of at least two antigens comprising using the composition of claim 11 for detecting in an affinity-binding assay for immune cells specific for said synthetic antigen, clonal expansion of antigen specific immune cells in samples of cells obtained from a mammal immunized with an antigen of said collection of antigens, and comparing said clonal expansion with the clonal expansion of another mammal immunized with another antigen of said collection, and selecting from said collection an antigen with which clonal expansion was more extensive than clonal expansion observed with at least one other antigen from said collection, wherein said collection of antigens comprises any antigen according to claim 18-22.

13. A method according to claim 12, wherein said affinity-binding assay comprises an assay according to any one of claims 1-10 or a method according to any one of claims 42-45

14. A method according to claim 13, wherein said at least two binding molecules comprise as a binding part for said target molecule, a synthetic antigen of said collection of antigens, or a native antigen or homologue of said antigen.

15. A method according to claim 14, further comprising selecting antigen for which clonal expansion of antigen specific immune cells is more extensive than the clonal expansion with at least two other antigens

16. A method according to claim 14 or 15, further comprising evaluating whether said antigen specific immune cells are specific for a native antigen or homologue of said antigen.

17. A method for an affinity-binding assay for selecting antigen specific immune cells, comprising:
a. contacting a cell having at least four copies of a target molecule with at least two binding molecules specific for said target molecule, wherein a first of said binding molecules is associated with a first label and a second of said binding molecules is associated with a second label and;
b. detecting cells staining with each label and;
c. selecting cells binding both labels.

18. A synthetic antigen, inducing a cross-reactive immune response with a native protein, selectable with a method according to any of claims 12-16 for use as a vaccine.

19. An antigen according to claim 18, wherein said antigen comprises a dimer peptide.

20. An antigen according to claim 18 or 19, wherein said antigen comprises a tandem dimer peptide.

21. An antigen according to any of claims 18 to 20, comprising a mimotope of said antigen.

22. An antigen according to any of claims 18 to 21, wherein at least one amino acid is substituted by a D-amino acid and/or a L-alanine.

23. An antigen according to any of claims 18 to 22, wherein said peptide is derived from GnRH.

24. An antigen according to any of claims 18 to 22, wherein said peptide is derived from Gastrin.

25. An antigen according to any of claims 18 to 22, wherein said peptide is derived from hCG .

26. An antigen according to any of claims 18 to 22, wherein said peptide is derived from PTHrP.

27. Use of an antigen according to any of claims 18 to 26 for the preparation of a medicament.

28. Use of an antigen according to claim 23 for the preparation of a medicament for the treatment of prostate cancer and of female GnRH-related cancer.

29. Use of an antigen according to claim 23 for the preparation of a medicament for blocking fertility.

30. Use of an antigen according to claim 24 for the preparation of a medicament for the treatment of Gastrin-related colorectal cancer, and/ or gastric cancer.

31. Use of an antigen according to claim 25 for the preparation of a medicament for the treatment of hCG-related gynaecological, colorectal, seminoma testicular, bladder, liver, stomach, pancreas, lung, brain, and kidney cancers.

32. Use of an antigen according to claim 25 for the preparation of a medicament for the treatment of hCG-related ulcers, duke's disease, and cirrhosis.

33. Use of an antigen according to claim 26 for the preparation of a medicament for the treatment of PTHrP-related cancer.

34. Use of an antigen according to claim 25 for the preparation of a medicament for the treatment of PTHrP-related osteolytic bone disease.

35. Use of an antigen according to claim 25 for the preparation of a medicament for the treatment of PTHrP-related hypercalcaemia.

36. An isolated B-cell detected with the method according to claim 17, for the preparation of an antibody.

37. A hybridoma based on the B cell of claim 36

38. An isolated antibody produced by a B-cell according to claim 36 or a hybridoma according to claim 37.

39. An isolated antibody according to claim 38 for the preparation of a medicament.

40. A kit of parts comprising at least a first binding molecule associated with a first label and a second binding molecule associated with a second label, said binding molecules comprising at least two binding regions for the same target molecule.

41. Use of a kit of parts according to claim 40 for the selection of an antigen.

42. A method for detecting a particle having at least four copies of a target molecule comprising contacting said particle with a first and a second binding molecule having at least two binding sites for said target molecule, said first binding molecule coupled to a first label and said second binding molecule coupled to a second label, and detecting particles having both labels.

43. A method for detecting a particle according to claim 42, wherein said detecting comprises detecting of a combined signal from said first and said second binding molecule.

44. A method for detecting a particle according to claim 42, wherein said particle is derived from a mammal within 14 days after vaccination with a native antigen or homologue of said antigen.

45. A method for detecting a particle according to claim 42, wherein said first binding molecule and said second binding molecule are present at approximately equimolar amounts.
